# EUROPÄISCHE PATENTANMELDUNG

(11) **EP 2 072 974 A1**
(43) Veröffentlichungstag der Anmeldung: **24.06.2009**
(21) Anmeldenummer: 07123572.5
(22) Anmeldetag: 19.12.2007
(51) Int. Cl.: G01G 13/02, G01N 1/20

(54) **Laborgerät mit einer dosiergut-führungsvorrichtung**

(71) Anmelder: Mettler-Toledo AG, 8606 Greifensee (CH)
(72) Erfinder: Lüchinger, Paul, 8610, Uster (CH)

(57) **Zusammenfassung**

Ein Laborgerät mit einer Dosiervorrichtung zum Dosieren von pulver-, pasten- oder granulatförmigem Dosiergut in mindestens ein Zielgefäss ist mit einer Dosiergut-Führungsvorrichtung ausgestattet, welche zwischen einer Auslassöffnung der Dosiervorrichtung und dem Zielgefäss angeordnet ist. Die Dosiergut-Führungsvorrichtung umfasst ein Bewegungsmittel und mindestens eine am Bewegungsmittel angeordnete Trichterhalterung, die zur Aufnahme eines auswechselbaren Trichters dient, welcher im eingesetzten Zustand im betriebsbereiten Laborgerät eine sich in Schwerkraftrichtung verjüngende Form aufweist.

## Beschreibung

Die Erfindung betrifft ein Laborgerät mit einer Dosiervorrichtung zum Dosieren von pulverförmigem, pastenförmigem oder granulatförmigem Dosiergut in ein Zielgefäss.

Laborgeräte mit Dosiervorrichtungen der vorgenannten Art finden insbesondere beim Dosieren kleiner Mengen, beispielsweise toxischer Substanzen, mit hoher Präzision in Zielgefässe Verwendung. Häufig sind solche Zielgefässe auf einer Waage platziert, um die Menge der aus der Dosiervorrichtung ausgetragenen Substanz zu verwiegen, so dass sie anschliessend bestimmungsgemäss weiter verarbeitet werden kann.

Die zu dosierende Substanz beziehungsweise das zu dosierende Dosiergut befindet sich beispielsweise in einem Entnahmegefäss, welches einen Dosierkopf aufweist. Dabei ist es wünschenswert, das zu dosierende Dosiergut durch eine kleine Öffnung der Dosiervorrichtung auszutragen, so dass sie auch gezielt in ein Gefäss mit einem kleinen Öffnungsquerschnitt abgefüllt werden kann.

Geräte mit Dosiervorrichtungen für pulverförmiges Schüttgut, beispielsweise Farbstoffpulver sind bekannt und im Einsatz. So wird in der US 5,145,009 A eine Vorrichtung zum Dosieren beschrieben, die aus einem Entnahmegefäss mit einem verschliessbaren Auslass an seiner Unterseite besteht. Als Verschlusselement dient ein kegelförmiger, sich nach oben verjüngender und zum Öffnen einer Auslassöffnung vertikal nach unten verstellbarer Ventilkörper, der in seiner Offenstellung rotiert und Mittel zum Fördern des Gutes in Richtung der Auslassöffnung besitzt. Ferner wird das Entnahmegefäss von einer Antriebswelle durchdrungen, welche an der Oberseite des Entnahmegefässes über dieses hinausragt und mit einem Antrieb gekoppelt ist. Das zu befüllende Zielgefäss ruht während des Dosiervorganges auf einer Waage, deren Wägesignal an eine Prozessoreinheit in der Antriebsvorrichtung des Verschlusselements weitergeleitet wird. Durch die Erfassung der ausdosierten Menge Dosiergut mittels einer Waage, kann das Verschlusselement bei Erreichen des Zielgewichtes im richtigen Moment geschlossen werden.

Die beschriebene Einrichtung eignet sich weniger gut für das Dosieren von Schüttgut in Gefässe mit einem kleinen Öffnungsquerschnitt. Die sich nach oben verjüngende Struktur des Ventilkörpers sowie dessen Rotation ergeben eine Radial- bzw. Horizontalkomponente der Schüttgutpartikel am Auslass und verursachen demzufolge eine Streuung, die selbst über einen relativ breiten Öffnungsquerschnitt eines abzufüllenden Gefässes hinausreichen kann.

Aus technischen Gründen kann der Querschnitt der Auslassöffnung aber nicht beliebig reduziert werden, um noch in Zielgefässe mit kleinsten Öffnungsquerschnitten eindosieren zu können. Limitationen ergeben sich beispielsweise aus der Partikel- oder Korngrösse und den Fliesseigenschaften des zu dosierenden Dosierguts sowie der Ausgestaltung der Auslassöffnung und insbesondere der Ausgestaltung des Verschlusselements. Ferner sollte der Randbereich der Zielgefäss-Einfüllöffnung, beispielsweise der sogenannte Schliff, möglichst nicht mit Dosiergut verschmutzt werden, da gegebenenfalls die Einfüllöffnung nach dem Befüllen mit einem Verschlussstopfen aus Glas dicht verschlossen werden soll.

Aufgabe der Erfindung ist es daher, ein Laborgerät mit einer Dosiervorrichtung zur Verfügung zu stellen, mit welchem präzise dosierte Kleinstmengen von pulverpasten- oder granulatförmigem Dosiergut gezielt in ein Zielgefäss mit kleinem Öffnungsquerschnitt eingefüllt werden können.

Diese Aufgabe wird durch ein Laborgerät gemäss dem ersten Anspruch erfüllt.

Ein Laborgerät umfasst mindestens eine Dosiervorrichtung, womit pulver-, pasten- oder granulatförmiges Dosiergut in ein Zielgefäss dosiert werden können. Dabei kann die Dosiervorrichtung einer der oben beschriebenen bekannten Ausführungsformen entsprechen und beispielsweise ein Entnahmegefäss und einen Dosierkopf aufweisen. Am Dosierkopf ist üblicherweise an dessen tiefster Stelle, bezogen auf die Lage des Dosierkopfs im Betriebszustand, eine Auslassöffnung ausgebildet, durch welche Austrittsöffnung das im Entnahmegefäss enthaltene Dosiergut ausgetragen werden kann. Der Durchlassquerschnitt der Austrittsöffnung wird üblicherweise mittels eines Dosierkopfventils variiert.

Erfindungsgemäss weist das Laborgerät mindestens eine zur Dosiervorrichtung mechanisch unabhängig angeordnete Dosiergut-Führungsvorrichtung auf. Mechanisch unabhängig bedeutet, dass keine direkte, mechanische Verbindung zwischen der Dosiervorrichtung und der Dosiergut- Führungsvorrichtung besteht. So lässt sich beispielsweise die Dosiervorrichtung auswechseln, ohne dass die Dosiergut-Führungsvorrichtung entfernt werden muss. Ferner impliziert dies auch, dass die Dosiergut-Führungsvorrichtung nicht an der Dosiervorrichtung befestigt ist. Dadurch ist ferner sichergestellt, dass Bewegungen der Dosiergut-Führungsvorrichtung nicht auf die Dosiervorrichtung übertragen werden. Jedoch ist die Dosiergut-Führungsvorrichtung wie auch die Dosiervorrichtung Teil des Laborgeräts, folglich sind beide Vorrichtungen zumindest gegen den Untergrund des Laborgeräts abgestützt und daher indirekt mechanisch miteinander verbunden.

Die Dosiergut-Führungsvorrichtung ist zwischen der Auslassöffnung der mindestens einen Dosiervorrichtung und dem Zielgefäss angeordnet. Selbstverständlich kann das Laborgerät mehrere Dosiervorrichtungen aufweisen und es kann jeder Dosiervorrichtung eine Dosiergut-Führungsvorrichtung zugeordnet sein. Die Dosiergut-Führungsvorrichtung beinhaltet ein Bewegungsmittel und mindestens eine am Bewegungsmittel angeordnete Trichterhalterung. Die Trichterhalterung ist als Halterung für einen auswechselbaren Trichter ausgestaltet, welcher in seiner Arbeitsstellung in der Trichterhalterung eine sich in Schwerkraftrichtung verjüngende Form hat, wodurch die Auslassöffnung der Dosiervorrichtung verlassendes Dosiergut zu einem engen Strom gebündelt und in die Öffnung des Zielgefässes geführt wird. Der Trichter ist ausschliesslich mit der mindestens einen Trichterhalterung verbindbar. Zusammen mit der mechanischen Unabhängigkeit der Dosiergut-Führungsvorrichtung zur Dosiervorrichtung wird dadurch die rasche Auswechselbarkeit des Trichters ermöglicht.

Das Bewegungsmittel dient dazu, die Trichterhalterung und den darin eingesetzten Trichter während des Dosiervorganges in eine oszillierende Bewegung zu versetzen. Dadurch wird der Trichter beispielsweise einer linearen und/oder rotatorischen Vibrations- oder Schüttelbewegung unterworfen, welche den Fluss des Dosiergutes durch den Trichter fördert und der Ansammlung von Dosiergut an der Trichterwand oder in der Ausflussmündung des Trichters entgegenwirkt. Die lineare Oszillation, beziehungsweise die Drehachse der rotatorischen Oszillation, kann horizontal, vertikal oder in beliebig geneigter Richtung orientiert sein. Als Antrieb für die Vibrations- oder Schüttelbewegung kommen beispielsweise ein piezoelektrischer Ultraschallgenerator oder eine von einem Elektromotor angetriebene Exzentermasse in Frage.

Aus der vorangehenden Beschreibung geht implizit hervor, dass der Trichter im Betriebszustand mit geeigneten Befestigungsmitteln fest mit der Trichterhalterung verbunden sein muss, damit sich die Bewegungen des Bewegungsmittels auf den Trichter übertragen lassen. Vorzugsweise werden zu diesem Zwecke Klemm-, Spann-, oder Einschnappsysteme verwendet, welche ein rasches Austauschen des Trichters ermöglichen. Der Trichter weist vorzugsweise geeignete Vorsprünge an dessen Aussenseite auf, welche mit den Befestigungsmitteln übereinstimmen und eine formschlüssige Verbindung mit den Befestigungsmitteln ergeben.

Die rasche Auswechselbarkeit des Trichters, unabhängig von der Dosiervorrichtung ist deshalb wichtig, damit gegebenenfalls Dosiergutrückstände zusammen mit dem Trichter entsorgt und somit nicht beim nächsten Dosiervorgang einem andern Dosiergut beigemischt werden. Vorzugsweise ist deshalb der Trichter als Verbrauchsmaterial konzipiert, aus einem kostengünstigen Material gefertigt, beispielsweise aus Kunststoffen wie PTFE oder Polyethylen, aber auch aus keramischen Materialien oder Metall.

Ferner kann auch mitten im Dosiervorgang den Trichter verstopfendes Dosiergut entfernt werden, indem der Dosiervorgang unterbrochen, der verstopfte Trichter entfernt und dieser durch einen neuen Trichter ersetzt wird. Mit dem neuen Trichter kann der Dosiervorgang wieder aufgenommen werden, wobei zu Beginn dieser Nachdosierung die bereits im Zielgefäss eindosierte Dosiergutmenge erfasst wird, die noch zu dosierende Differenzmenge zur Zielmenge berechnet wird und diese Differenzmenge in das Zielgefäss dosiert wird.

Am Trichter anhaftende Dosiergutrückstände können sich ausserdem in der Endphase eines Dosiervorgangs störend auswirken, da das Dosierkopfventil beispielsweise in Abhängigkeit des zunehmenden Gewichts des Zielbehälters auf der Waage gesteuert wird und die im Trichter haftenden Dosiergutrückstände nicht erfasst werden. Insbesondere beim präzisen Dosieren von Kleinstmengen kann dies dazu führen, dass die ausdosierte Menge nicht innerhalb der geforderten Bandbreite der Zielmenge ist. Das letztgenannte Problem könnte mit einer weiteren Wägezelle gelöst werden, mit welcher auch der Trichter während des Dosiervorganges laufend gewogen wird.

Selbstverständlich kann zusätzlich auch das Gewicht der Dosiervorrichtung erfasst werden. Die Abnahme deren Masse müsste die Summe der Masse im Trichter und der Masse im Zielgefäss entsprechen. Diese Ausgestaltung könnte dazu dienen, den Verlust von Dosiergut genau zu erfassen. Je nach Art des Dosierguts, beispielsweise bei toxischen Substanzen, könnte durch dieses System der Benutzer gewarnt werden.

Vorzugsweise ist das Laborgerät mit einem Nachfüllmagazin für neue Trichter und mit einer Entsorgungsstation für gebrauchte Trichter ausgerüstet. Dadurch wird ein leichtes Auswechseln des Trichters nach jedem Dosiervorgang ermöglicht, was besonders dann wichtig ist, wenn auch der Dosierkopf ausgewechselt wird und ein anderes Dosiergut dosiert werden soll.

Bei der vorgenannten Ausführung des erfindungsgemässen Laborgerätes ist es vorteilhaft, wenn die Trichterhalterung für den Trichter zwischen mindestens drei Positionen bewegbar ist, wobei die erste Position mit dem Nachfüllmagazin, die zweite Position mit der Dosiervorrichtung und die dritte Position mit der Entsorgungsstation korrespondiert. Beispielsweise kann die Verschiebung zwischen den drei Positionen durch Schwenken oder Drehen um eine Achse realisiert werden. Selbstverständlich sind auch lineare Verschiebungen möglich.

Dabei kann die Trichterhalterung ferner mit einer automatischen Abwurfvorrichtung versehen sein, welche so gestaltet ist, dass nach der Verschiebung der Trichterhalterung in die dritte Position der Trichter automatisch in die Entsorgungsstation befördert wird.

Speziell bei pappigem und nicht frei rieselndem Dosiergut ist es wichtig, dass der Trichter während eines Dosiervorganges überwacht wird und dass bei übermässiger Materialablagerung im Trichter oder gar einer Verstopfung des Trichters der Dosiervorgang gestoppt wird. Um diese Überwachung zu automatisieren, kann die Trichterhalterung beispielsweise mit einem Ultraschallsensor, einem Infrarotsensor, einem Lichtschrankensensor oder auch mit einer Wägezelle ausgerüstet sein.

Eine weitere Ausgestaltung des erfindungsgemässen Laborgeräts umfasst zudem eine erste Wägezelle mit einem Lastaufnehmer für ein Zielgefäss, eine zweite Wägezelle zur Erfassung des Trichtergewichts, sowie eine Prozessoreinheit zur Verarbeitung der von der ersten Wägezelle und von der zweiten Wägezelle erzeugten Wägesignale, welche zur Steuerung der Austragungsleistung der Dosiervorrichtung verwendet werden.

Bei allen Ausführungsformen der Erfindung ist der Trichter vorteilhafterweise als ein sich konisch verjüngendes Rohr ausgebildet, welches einen grossen Eintrittsquerschnitt und einen kleinen Austrittsquerschnitt aufweist, wobei der zwischen der Trichterwand und der Konusachse des Trichters eingeschlossene Winkel vorzugsweise nicht mehr als 10º beträgt. Bei gegebenem Eintrittsquerschnitt und Austrittsquerschnitt wird der Trichter umso länger, je steiler die Trichterwand angeordnet ist, doch verringert sich dabei die Gefahr, dass Dosiergut im Trichter stecken bleibt oder durch die Vibrationen gegen oben geschleudert wird.

Ebenfalls zur Verhinderung, dass Dosiergut durch die Vibrationen zurückgeschleudert und über den Rand des Eintrittsquerschnittes geworfen wird, kann im Bereich des Eintrittsquerschnitts des Trichters ein Prallring angeordnet sein. Am Austrittsquerschnitt des Trichters kann ferner ein Endrohr angeordnet sein. Dieses kann wesentlich länger als der Trichter ausgebildet sein, so dass das Endrohr tief in das Zielgefäss hineinragen kann.

Selbstverständlich kann der Trichter eine beliebige Querschnittform aufweisen. Vorzugsweise ist dieser Querschnitt dem Öffnungsquerschnitt des Zielgefässes und der Schwingungsamplitude des Bewegungsmittels angepasst.

Ferner kann zur Reduzierung der Haftung von Dosiergut an der Innenfläche des Trichters diese Innenfläche eine spezielle Oberflächenbehandlung oder Beschichtung, beispielsweise eine Nanopartikel- Beschichtung aufweisen. Weitere Möglichkeiten, die Rutscheigenschaften des Dosiergutes zu verbessern bestehen darin, dass die Innenfläche des Trichters spezielle, auf die Schwingungen des Bewegungsmittels abgestimmte Oberflächenstrukturen aufweist. Beispielsweise könnte die Trichter-Innenseite eine fischschuppenartige Oberflächenstruktur aufweisen, wobei die Dimensionen der einzelnen Schuppen auf die Amplitude, die Bewegungsrichtung und die Frequenz des Bewegungsmittels abgestimmt sind, so dass das Dosiergut im Randbereich des Trichters infolge der Schwingungen und der strukturierten Oberfläche ausschliesslich Beschleunigungen zum Zielgefäss hin erfährt und der Gleitwiderstand der Oberfläche reduziert wird.

Beim Dosieren von pulverförmigem, pastenförmigem oder granulatförmigem Dosiergut in ein Zielgefäss mit einem erfindungsgemässen Laborgerät, kann vorzugsweise ein Verfahren mit den folgenden Schritten angewendet werden:
- Ein Trichter wird in die Trichterhalterung eingesetzt.
- Der Eintrittsquerschnitt des Trichters wird zur Auslassöffnung der Dosiervorrichtung ausgerichtet und das Zielgefäss wird mit seiner Einfüllöffnung zum Austrittsquerschnitt des Trichters in Übereinstimmung gebracht.
- Die Ausgabe von Dosiergut aus der Dosiervorrichtung wird gestartet, wobei gleichzeitig das Bewegungsmittel in Betrieb gesetzt wird.
- Die Ausgabe von Dosiergut aus der Dosiervorrichtung erfolgt solange, bis das vom Zielgefäss empfangene Dosiergut eine vorgegebene Zielmenge erreicht hat.
- Nach dem Abschluss des Dosiervorganges wird der Trichter aus der Trichterhalterung entfernt.

Bei der oben beschriebenen Variante eines erfindungsgemässen Laborgerätes, welches nebst der Wägezelle für das Zielgefäss eine zweite Wägezelle zur Erfassung des Trichtergewichtes aufweist, wird das vorstehende Verfahren mit Vorteil in dem Sinne angepasst, dass die Ausgabe von Dosiergut aus der Dosiervorrichtung solange erfolgt, bis die Gewichtssumme des vom Zielgefäss empfangenen Dosierguts und des noch im Trichter befindlichen Dosierguts ein vorgegebenes Zielgewicht erreicht hat, wobei der Trichter anschliessend noch solange bewegt, beziehungsweise vibriert wird, bis möglichst der gesamte Rest des Dosiergutes aus dem Trichter in das Zielgefäss befördert worden ist. Falls noch Rückstände im Trichter verbleiben, so dass die im Zielgefäss angekommene Dosiergutmenge das Zielgewicht nicht ganz erreicht, wird die Differenz gegebenenfalls durch Nachdosieren ausgeglichen.

Das erfindungsgemässe Laborgerät wird im Folgenden anhand von Beispielen und mit Bezugnahme auf die Zeichnungen näher erläutert. Darin zeigen:
- Figur 1: eine schematisch vereinfachte Darstellung eines erfindungsgemässen Laborgeräts in seinem Betriebszustand, mit einer Dosiervorrichtung, einer Dosiergut-Führungsvorrichtung und einem Zielgefäss;
- Figur 2: eine erweiterte Ausführungsform des Laborgeräts von Figur 1, mit einem Nachfüllmagazin für neue Trichter, einer Entsorgungsstation für gebrauchte Trichter und einer Vorrichtung zum Abwurf des gebrauchten Trichters;
- Figur 3: eine schematische Darstellung des Signalflusses zur Steuerung eines Dosiervorgangs in einem erfindungsgemässen Laborgerät;
- Figur 4: eine schematische Darstellung eines Trichters mit einem Prallring; und
- Figur 5: eine schematische Darstellung eines Bewegungsmittels mit mehreren, am selben Trichter angreifenden Trichterhalterungen.

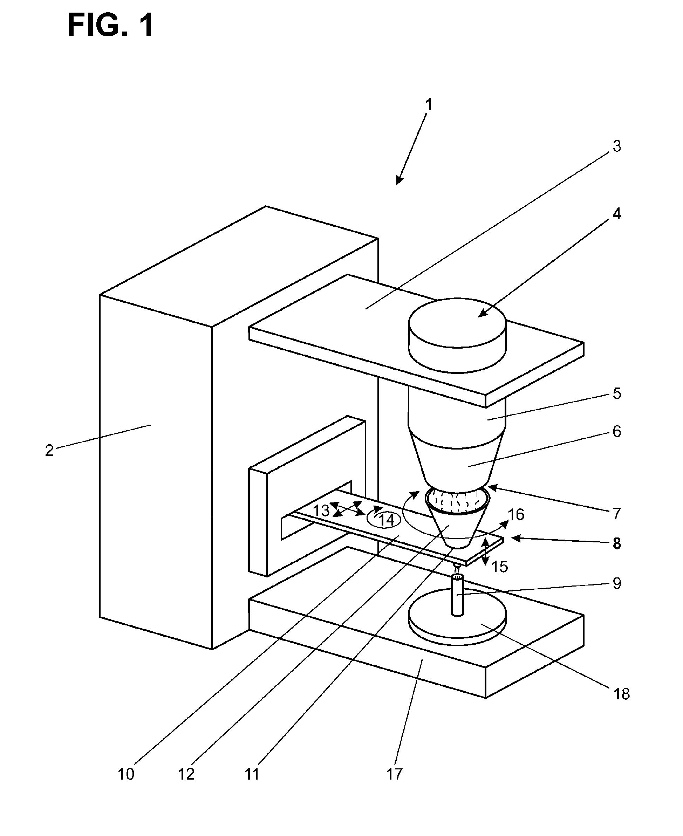

Das in Figur 1 dargestellte erfindungsgemässe Laborgerät 1 umfasst ein Gehäuse 2 mit einem Tragarm 3 in welchem eine auswechselbare Dosiervorrichtung 4 eingesetzt ist, welche ihrerseits einen Entnahmebehälter 5 für das Dosiergut und einen Dosierkopf 6 mit einer Auslassöffnung 7 aufweist. Die Auslassöffnung 7 ist in der Zeichnung nicht direkt sichtbar. Das Laborgerät 1 und die Dosiervorrichtung 4 sind mit bekannten und deshalb nicht abgebildeten Mitteln versehen, mit denen das hier schematisch angedeutete Ausströmen des Dosierguts aus der Auslassöffnung 7 gesteuert werden kann.

Direkt unterhalb der Auslassöffnung 7 ist die Dosiergut-Führungsvorrichtung 8 angeordnet. Diese besteht aus einem Bewegungsmittel 10 in der Form eines Trägers mit einer Trichterhalterung 11, welche als Aufnahme für einen auswechselbaren Trichter 12 dient. Das Bewegungsmittel 10 mit dem Trichter 12 kann durch einen an sich bekannten und deshalb nicht abgebildeten Bewegungsgenerator, beispielsweise einen piezoelektrischen Vibrator oder einen Elektromotor mit Exzentermasse in eine Vibration oder Schüttelbewegung versetzt werden, welche dem Anhaften von Dosiergut am Trichter 12 entgegenwirkt und einen leichtgängigen Rieselfluss des Dosiergutes durch den Trichter 12 fördert. Verschiedene mögliche Bewegungsformen des Bewegungsmittels 10 beziehungsweise Trägers sind symbolisch durch Pfeile dargestellt, beispielsweise durch
- seitliche und/oder longitudinal oszillierende Bewegungen, symbolisch dargestellt durch die gekreuzten Pfeile 13,
- kreisförmige Bewegungen in einer Ebene, symbolisch dargestellt durch den Drehpfeil 14,
- vertikale oszillierende Bewegungen, symbolisch dargestellt durch den Doppelpfeil 15, oder
- oszillierende Drehbewegungen des Trichters um seine Mittellängsachse, symbolisch dargestellt durch den Doppeldrehpfeil 16.

Direkt unterhalb der Auslassöffnung des Trichters 12 befindet sich das Zielgefäss 9, welches auf dem Lastaufnehmer 18 einer ersten Wägezelle 17 aufgesetzt ist.

Wie Figur 1 anschaulich darstellt, dient der Trichter 12 dazu, den aus der Auslassöffnung 7 des Dosierkopfes 6 fliessenden, breit gestreuten Strom des Dosiergutes zu verengen und zur Öffnung des Zielgefässes 9 zu lenken.

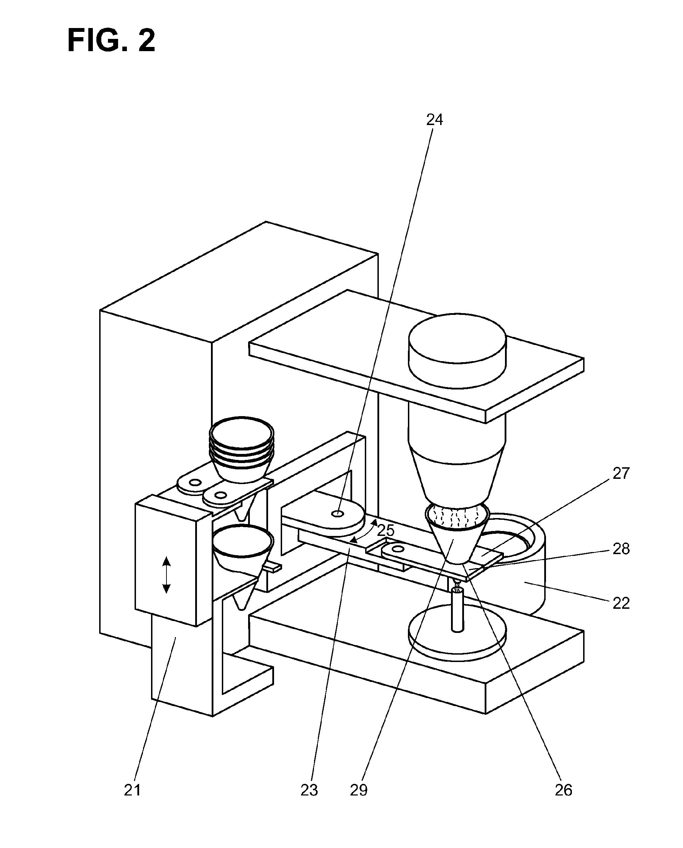

Zusätzlich zur in Figur 1 gezeigten Anordnung sind in Figur 2 ein Nachfüllmagazin 21 für neue Trichter 29 sowie eine Entsorgungsstation 22 für gebrauchte Trichter 29 schematisch dargestellt. Das Nachfüllmagazin 21 ist derart ausgelegt, dass es eine Trichterabgabestelle aufweist, die jeweils automatisch mit einem neuen Trichter 29 aus einem Trichtervorrat bestückt wird, wenn ein Trichter 29 diese verlässt.

Wie durch den Doppeldrehpfeil 25 schematisch angedeutet, ist das Bewegungsmittel 23 in Form eines Trägerarms in Figur 2 in einer Horizontalebene um das Lager 24 schwenkbar gelagert. Ausserdem ist das Bewegungsmittel 23 für den automatischen Abwurf des Trichters 29 in die Entsorgungsstation 22 und das Aufnehmen eines neuen Trichters aus dem Nachfüllmagazin 21, beziehungsweise aus dessen Trichterabgabestelle ausgerüstet. Diese Funktion des Bewegungsmittels 23 ist in Figur 2 schematisch dargestellt, indem die Trichterhalterung 26, welche den Trichter 29 aufnimmt, von einem integralen Fortsatz 27 des Bewegungsmittels 23 und einem vom Fortsatz 27 wegschwenkbaren Finger 28 umfasst wird. Zum Abwurf eines gebrauchten Trichters 29 wird das Bewegungsmittel 23 zur Entsorgungsstation 22 geschwenkt, der Finger 28 wird vom Fortsatz 27 weggespreizt, und der Trichter fällt in die Entsorgungsstation 22. Zum Aufnehmen eines neuen Trichters 29 wird das Bewegungsmittel 23 zum Nachfüllmagazin 21 hin geschwenkt.

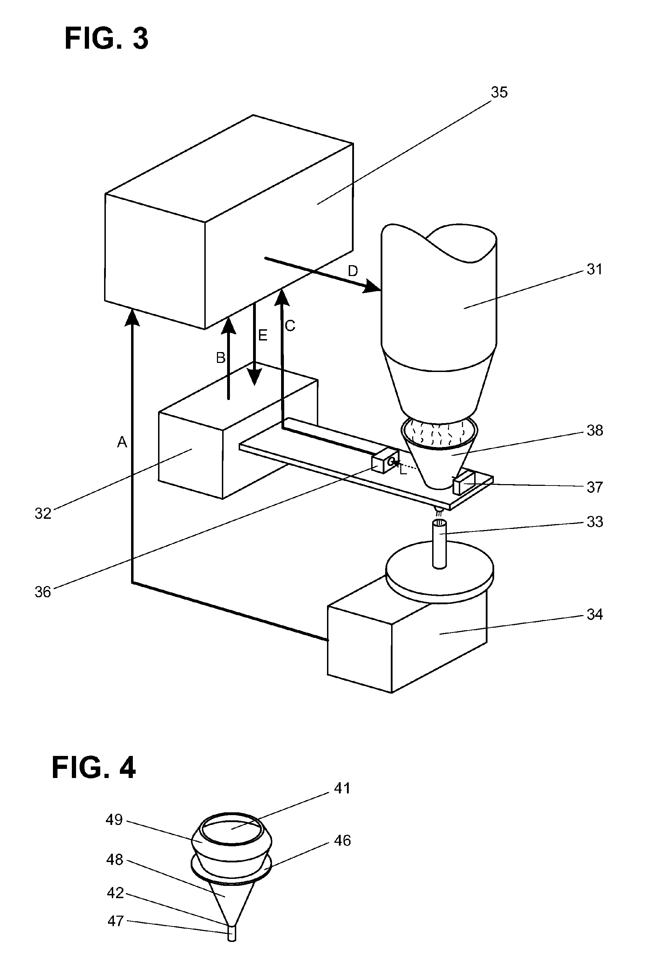

Figur 3 zeigt schematisch und beispielhaft den Signalfluss der Steuer- und Regelkreise zum Steuern der Dosiervorrichtung 31 und der Dosiergut-Führungsvorrichtung 32. Das Gewicht des Zielgefässes 33, beziehungsweise das Nettogewicht des darin befindlichen Dosierguts wird von der Wägezelle 34 laufend an die Prozessoreinheit 35 übermittelt, symbolisch durch den Pfeil A dargestellt. Ausserdem empfängt die Prozessoreinheit 35 von der Dosiergut-Führungsvorrichtung 32 das Signal B, welches das Gewicht von gegebenenfalls im Trichter 38 befindlichem Dosiergut darstellt. Eine Lichtschranke 36, 37 wirkt als Sicherheitsabschalter für die Dosiervorrichtung 31, wobei der Trichter 38 zur Realisierung dieses Konzepts aus einem lichtdurchlässigen Material besteht. Solange das Dosiergut frei durch den Trichter 38 fliesst, empfängt der Lichtsensor 36 den von der Lichtquelle 37 durch den unteren Bereich des Trichters 38 gesandten Lichtstrahl L. Der Lichtstrahl L als analoges Signal beinhaltet zwei Informationen. Erstens wird bei abnehmender Intensität durch den Lichtsensor 36 erfasst, dass sich Dosiergut an der Trichterwand oder im Trichter 38 ansammelt. Zweitens kann aber auch die Flussdichte des den Trichter 38 durchströmenden Dosierguts erfasst und ausgewertet werden.

Wenn sich der Trichter beispielsweise bei pappigem Dosiergut zu verstopfen beginnt, wird der Lichtstrahl L blockiert, was der Prozessoreinheit 35 durch das Signal C gemeldet wird. Aufgrund der empfangenen Signale A, B, C steuert die Prozessoreinheit 35 einerseits die Dosiervorrichtung 31 durch das Signal D und andrerseits die Dosiergut-Führungsvorrichtung 32 durch das Signal E. Das Signal D repräsentiert hier die gesamte digitale und/oder analoge Information, durch welche das Starten und Stoppen sowie gegebenenfalls die variable Austragsrate der Dosiervorrichtung 31 gesteuert wird. Das Signal E dient zum An- und Abschalten und gegebenenfalls zur Steuerung der Frequenz und/oder Amplitude der Vibration oder Schüttelbewegung, mit welcher die Dosiergut-Führungsvorrichtung 32 auf den Trichter 38 einwirkt.

In Figur 4 ist ein Trichter 48 dargestellt, welcher im Bereich seines Eintrittquerschnitts 41 ein Prallring 49 aufweist. Dieser verhindert, dass sich infolge der Vibrationen Dosiergut entgegen der Richtung der Schwerkraft über den Eintrittquerschnitt 41 hinaus bewegen kann. Am Austrittquerschnitt 42 des Trichters 48 kann zudem ein zylindrisches Endrohr 47 angeordnet sein, durch welches Endrohr 47 das Dosiergut an der Verschlusszone, beispielsweise an einer konisch geschliffenen Innenpartie eines Zielgefässes vorbeigeführt werden kann. Selbstverständlich kann das Endrohr 47 wesentlich Länger ausgestaltet sein als die Länge des Trichters 48. Damit der Trichter 48 beispielsweise mittels eines Roboters in eine Trichterhalterung eingesetzt werden kann, können an der Trichter-Aussenseite Halte- und Greifmöglichkeiten ausgebildet sein. Diese sind in Figur 4 schematisch durch einen Bund 46 dargestellt. An diesen Bund 46 können auch die an der Trichterhalterung angeordneten Befestigungsmittel angreifen.

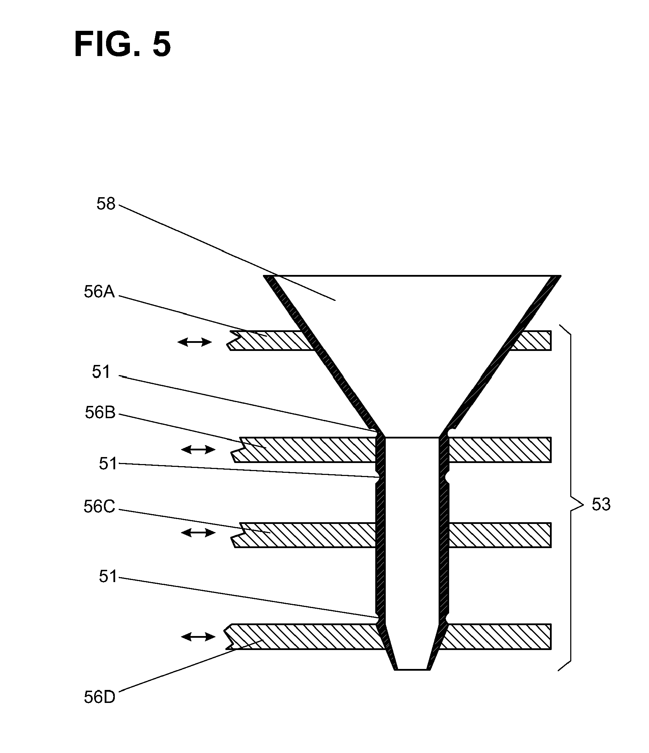

Figur 5 zeigt eine schematische Darstellung eines Bewegungsmittels 53 mit mehreren, am selben Trichter 58 angreifenden Trichterhalterungen 56A, 56B, 56C, 56D. Die Trichterhalterungen 56A, 56B, 56C, 56D sind übereinander angeordnet, wobei die Bohrungen zur Aufnahme des Trichters 58 derart ausgerichtet sind, dass sich von oben ein Trichter in die Trichterhalterungen 56A, 56B, 56C, 56D einsetzen lässt. Die einzelnen Trichterhalterungen 56A, 56B, 56C, 56D sind wie durch die Doppelpfeile angedeutet, individuell zueinander bewegbar. Dadurch können mit einer geeigneten mechanischen oder elektrischen Steuerung beliebige Bewegungsmuster, beispielsweise in der Längsrichtung des Trichters 58 laufende Wellenbewegungen, erzeugt werden.

Damit solche Bewegungsmöglichkeiten überhaupt dienlich sind, sollte der Trichter vorzugsweise aus einem flexiblen Material gefertigt sein. Nur so können sich die sehr kurzhubigen Bewegungen auf die einzelnen Abschnitte des Trichters 58 übertragen. Bei steiferen Materialien ist es von Vorteil, wenn der Trichter zusätzlich mit Dünnstellen 51 versehen ist.

Obwohl die Erfindung durch die Darstellung spezifischer Ausführungsbeispiele beschrieben worden ist, ist es offensichtlich, dass zahlreiche weitere Ausführungsvarianten in Kenntnis der vorliegenden Erfindung geschaffen werden können, beispielsweise indem die Merkmale der einzelnen Ausführungsbeispiele miteinander kombiniert und/oder einzelne Funktionseinheiten der Ausführungsbeispiele ausgetauscht werden. Insbesondere sind weitere Ausführungen denkbar, die den Erfindungsgegenstand beinhalten, beispielsweise wenn das Laborgerät in automatisierter Ausführung Bestandteil einer grösseren Vorrichtung ist.

### Bezugszeichenliste

- 1: Laborgerät
- 2: Gehäuse
- 3: Tragarm
- 31,4: Dosiervorrichtung
- 5: Entnahmebehälter
- 6: Dosierkopf
- 7: Auslassöffnung
- 32, 8: Dosiergut-Führungsvorrichtung
- 33, 9: Zielgefäss
- 53, 23, 10: Bewegungsmittel
- 56D, 56C, 56B, 56A, 26, 11: Trichterhalterung
- 58, 48, 38, 29, 12: Trichter
- 13: gekreuzte Pfeile
- 14: Drehpfeil
- 15: vertikaler Doppelpfeil
- 16: Doppeldrehpfeil
- 34, 17: Wägezelle
- 18: Lastaufnehmer
- 21: Nachfüllmagazin für neue Trichter
- 22: Entsorgungsstation für gebrauchte Trichter
- 24: Schwenklager
- 25: Doppeldrehpfeil
- 27: Fortsatz
- 28: abspreizbarer Finger
- 35: Prozessoreinheit
- 36: Lichtsensor
- 37: Lichtquelle
- 41: Eintrittquerschnitt
- 42: Austrittquerschnitt
- 46: Bund
- 47: Endrohr
- 49: Prallring
- 51: Dünnstelle
- A,B,C,D,E: analoge und/oder digitale Signale
- L: Lichtstrahl

## Patentansprüche

1. Laborgerät (1) mit mindestens einer Dosiervorrichtung (4, 31) zum Dosieren von pulver-, pasten- oder granulatförmigem Dosiergut in mindestens ein Zielgefäss (9, 33), **dadurch gekennzeichnet, dass** das Laborgerät (1) für mindestens eine Dosiervorrichtung (4, 31) eine zur Dosiervorrichtung (4,31) mechanisch unabhängig angeordnete Dosiergut- Führungsvorrichtung (8, 32) aufweist, welche Dosiergut- Führungsvorrichtung (8, 32) zwischen einer Auslassöffnung (7) der Dosiervorrichtung (4, 31) und dem Zielgefäss (9, 33) angeordnet ist, wobei die Dosiergut- Führungsvorrichtung (8, 32) ein Bewegungsmittel (10, 23, 53) und mindestens eine am Bewegungsmittel (10, 23, 53) angeordnete Trichterhalterung (11, 26, 56A, 56B, 56C, 56D) beinhaltet und wobei ein, bei betriebsbereitem Laborgerät (1) in Schwerkraftrichtung sich verjüngender, auswechselbarer Trichter (12, 29, 38, 48, 58) ausschliesslich mit der mindestens einen Trichterhalterung (11, 26, 56A, 56B, 56C, 56D) verbindbar ist.

2. Laborgerät (1) nach Anspruch 1, **dadurch gekennzeichnet, dass** ferner ein Nachfüllmagazin (21) für unbenutzte Trichter (12, 29, 38, 48, 58) und eine Entsorgungsstation (22) für benutzte Trichter (12, 29, 38, 48, 58) vorhanden sind.

3. Laborgerät (1) nach Anspruch 2, **dadurch gekennzeichnet, dass** die mindestens eine Trichterhalterung (11, 26, 56A, 56B, 56C, 56D) zwischen mindestens drei Positionen um eine Achse schwenkbar oder drehbar ausgebildet ist, wobei die erste Position mit dem Nachfüllmagazin (21), die zweite Position mit der Dosiervorrichtung (4, 31) und die dritte Position mit der Entsorgungsstation (22) korrespondiert.

4. Laborgerät (1) nach einem der Ansprüche 1 bis 3, **dadurch gekennzeichnet, dass** die mindestens eine Trichterhalterung (11, 26, 56A, 56B, 56C, 56D) eine automatische Abwurfvorrichtung (27, 28) aufweist.

5. Laborgerät (1) nach einem der Ansprüche 1 bis 4, **dadurch gekennzeichnet, dass** die mindestens eine Trichterhalterung (11, 26, 56A, 56B, 56C, 56D) eine Füllstandsüberwachungsvorrichtung (36, 37) zur Messung und Überwachung des im Trichter (12, 29, 38, 48, 58) während des Betriebes sich anhäufenden Dosierguts aufweist.

6. Laborgerät (1) nach einem der Ansprüche 1 bis 5, **dadurch gekennzeichnet, dass** mit dem Bewegungsmittel (10, 23, 53) auf den Trichter (12, 29, 38, 48, 58) einwirkende lineare (13, 15) oder kreisförmige (14) oder um Mittellängsachse des Trichters (12, 29, 38, 48, 58) oszillierende (16) Schüttelbewegungen, Vibrationen oder Ultraschallschwingungen erzeugbar sind.

7. Laborgerät (1) nach einem der Ansprüche 1 bis 6, **dadurch gekennzeichnet, dass** das Laborgerät (1) mindestens eine erste Wägezelle (34) mit einem Lastaufnehmer (18) für ein Zielgefäss (9, 33) und mindestens eine, mit der Dosiergut-Führungsvorrichtung (8, 32) verbundene und der Erfassung des Gewichts des Trichters (12, 29, 38, 48, 58) dienende, zweite Wägezelle aufweist und mindestens eine Prozessoreinheit (35) zur Verarbeitung der von der ersten Wägezelle (34) und von der zweiten Wägezelle erzeugten Wägesignale (A, B) zur Steuerung der Austragungsleistung der Dosiervorrichtung (4, 31) vorhanden ist.

8. Trichter (12, 29, 38, 48, 58), verbindbar mit einer Trichterhalterung (11, 26, 56A, 56B, 56C, 56D) eines Laborgeräts (1) nach einem der Ansprüche 1 bis 7, **dadurch gekennzeichnet, dass** der Trichter (12, 29, 38, 48, 58) rohrförmig ausgebildet ist, einen grossen Eintrittquerschnitt (41) und einen kleinen Austrittquerschnitt (42) aufweist und dass der zwischen der Trichterwand und der Mittellängsachse des rohrförmigen Trichters (12, 29, 38, 48, 58) eingeschlossene Winkel kleiner oder gleich 10° ist.

9. Trichter (12, 29, 38, 48, 58), nach Anspruch 8, **dadurch gekennzeichnet, dass** im Bereich des Eintrittquerschnitts (41) mindestens ein Prallring (49) und/oder am Austrittquerschnitt (42) ein zylindrisches Endrohr (47) angeordnet ist.

10. Trichter (12, 29, 38, 48, 58), nach Anspruch 8 oder 9, **dadurch gekennzeichnet, dass** die Innenseite des Trichters (12, 29, 38, 48, 58) eine die Haftung von Dosiergut reduzierende Oberflächenbehandlung und/oder eine auf die Bewegungen des Bewegungsmittels (10, 23, 53) abgestimmte Oberflächenstruktur aufweist.

11. Verfahren zum Dosieren von pulverförmigem, pastenförmigem oder granulatförmigem Dosiergut in ein Zielgefäss (9, 33) mit einem Laborgerät (1) nach einem der Ansprüche 1 bis 6, **dadurch gekennzeichnet, dass**
- in einem ersten Schritt die mindestens eine Trichterhalterung (11, 26, 56A, 56B, 56C, 56D) mit einem Trichter (12, 29, 38, 48, 58), nach einem der Ansprüche 8 bis 10 verbunden wird,
- in einem zweiten Schritt der Eintrittquerschnitt (41) des Trichters (12, 29, 38, 48, 58), mit der Auslassöffnung (7) der Dosiervorrichtung (4, 31) und die Einfüllöffnung eines Zielgefässes (9, 33) mit dem Austrittquerschnitt (42) des Trichters (12, 29, 38, 48, 58), in Übereinstimmung gebracht wird,
- in einem dritten Schritt die Dosiervorrichtung (4, 31) und das Bewegungsmittel (10, 23, 53) aktiviert werden und somit Dosiergut aus der Dosiervorrichtung (4, 31) ausdosiert wird, und
- nach dem Abschluss des Dosiervorganges in einem vierten Schritt der Trichter (12, 29, 38, 48, 58), von der Trichterhalterung (11, 26, 56A, 56B, 56C, 56D) entfernt wird.

12. Verfahren zum Dosieren von pulverförmigem, pastenförmigem oder granulatförmigem Dosiergut in ein Zielgefäss (9, 33) mit einem Laborgerät (1) nach Anspruch 7, **dadurch gekennzeichnet, dass**
- in einem ersten Schritt die mindestens eine Trichterhalterung (11, 26, 56A, 56B, 56C, 56D) mit einem Trichter (12, 29, 38, 48, 58), nach einem der Ansprüche 8 bis 10 verbunden wird,
- in einem zweiten Schritt der Eintrittquerschnitt (41) des Trichters (12, 29, 38, 48, 58), mit der Auslassöffnung (7) der Dosiervorrichtung (4, 31) und die Einfüllöffnung eines Zielgefässes (9, 33) mit dem Austrittquerschnitt (42) des Trichters (12, 29, 38, 48, 58), in Übereinstimmung gebracht wird,
- in einem dritten Schritt die Dosiervorrichtung (4, 31) und das Bewegungsmittel (10, 23, 53) aktiviert werden und somit Dosiergut aus der Dosiervorrichtung (4, 31) ausdosiert wird, wobei die Ausgabe von Dosiergut aus der Dosiervorrichtung (4, 31) solange erfolgt, bis die Gewichtssumme des vom Zielgefäss (9, 33) empfangenen Dosierguts und des noch im Trichter (12, 29, 38, 48, 58), befindlichen Dosierguts ein vorgegebenes Zielgewicht erreicht hat und der Trichter (12, 29, 38, 48, 58), anschliessend solange bewegt wird, bis möglichst der gesamte Rest des Dosierguts aus dem Trichter (12, 29, 38, 48, 58), in das Zielgefäss (9, 33) befördert worden ist,
- falls noch Rückstände im Trichter (12, 29, 38, 48, 58), verbleiben und demzufolge die im Zielgefäss (9, 33) angekommene Dosiergutmenge das Zielgewicht nicht ganz erreicht, in einem vierten Schritt die Differenz durch Nachdosieren ausgeglichen wird, und
- nach dem Abschluss des Dosiervorganges in einem fünften Schritt der Trichter (12, 29, 38, 48, 58), von der Trichterhalterung (11, 26, 56A, 56B, 56C, 56D) entfernt wird.
